(19) Europäisches Patentamt
European Patent Office
Office européen des brevets

(11) Publication number: **0 118 867 A2**

# (12) EUROPEAN PATENT APPLICATION

(21) Application number: **84102369.0**

(22) Date of filing: **06.03.84**

(51) Int. Cl.³: **C 07 D 495/04**
C 07 D 333/38, A 61 K 31/335
//(C07D495/04, 333/00, 313/00)

(30) Priority: **10.03.83 US 473881**

(43) Date of publication of application:
**19.09.84 Bulletin 84/38**

(84) Designated Contracting States:
**AT BE CH DE FR GB IT LI NL**

(71) Applicant: **HOECHST-ROUSSEL PHARMACEUTICALS INCORPORATED**
**Route 202-206 North**
**Somerville New Jersey 08876(US)**

(72) Inventor: **Martin, Lawrence Leo**
**R.D. 3 Box 81, Concord Road**
**Lebanon New Jersey 08833(US)**

(72) Inventor: **Setescak, Linda Louise**
**102 Kimberly Road**
**Somerville New Jersey 08876(US)**

(74) Representative: **Meyer-Dulheuer, Karl-Hermann, Dr. et al,**
**HOECHST Aktiengesellschaft Zentrale Patentabteilung**
**Postfach 80 03 20**
**D-6230 Frankfurt/Main 80(DE)**

(54) **Substituted 4,10-dihydro-10-oxothieno benzoxepins, a method of preparing the same and intermediate thereof and their use as medicaments.**

(57) There are described substituted 4,10-dihydro-10-oxothienobenzoxepins of the formula

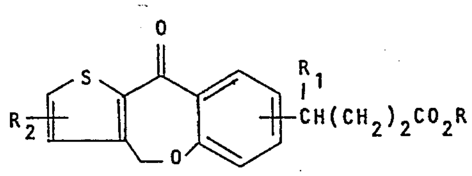

wherein R is hydrogen, loweralkyl,

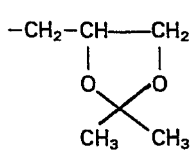

or $-CH_2CH(OH)CH_2OH$;

$R_1$ is hydrogen or loweralkyl; and $R_2$ is hydrogen or loweralkyl, which are useful as anti-inflammatory and analgetic agents, intermediate compounds therefor, and a method of preparing the same.

EP 0 118 867 A2

Croydon Printing Company Ltd.

0118867
HOE 83/S 007

This invention relates to novel 4,10-dihydro-10-oxothieno benzoxepins of the formula

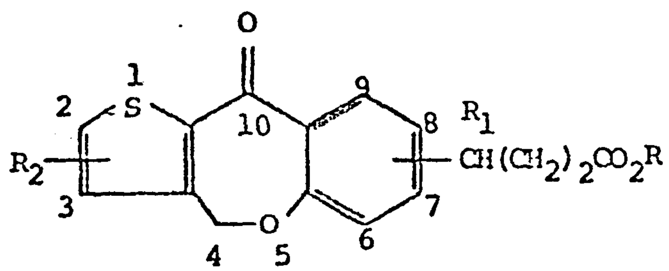

(I)

where R is hydrogen, loweralkyl, 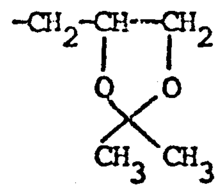 or $-CH_2CH(OH)CH_2OH$; $R_1$ is hydrogen or loweralkyl; and $R_2$ is hydrogen or loweralkyl, which are useful as anti-inflammatory and analgetic agents; to intermediate compounds therefor; and to methods of synthesizing the foregoing compounds.

To the best of our knowledge the compounds of the present invention have not heretofore been described or suggested.

One group of compounds of the present invention are compounds having the general formula I recited above. Particularly preferred compounds are those of compounds I where $R_2$ is hydrogen and $R_1$ is hydrogen or methyl.

In the above definitions and as used hereinafter, the term "lower" means the group it is describing contains 1 to 6 carbon atoms. The term "alkyl" refers to a straight or branched chain hydrocarbon containing no unsaturation e.g. methyl, ethyl, n-propyl, isopropyl, tertiary-butyl, etc.

Where an optical isomerism exists, a given chemical name or formula shall encompass all optical isomers, and mixtures thereof including the racemic mixture thereof throughout the specification and the appended claims.

The compounds of the present invention are prepared in the following manner. The groups R, $R_1$ and $R_2$ are as defined above unless indicated otherwise.

STEP A A compound of the formula

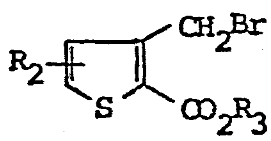

(II)

where $R_3$ is loweralkyl is reacted with a compound of the formula

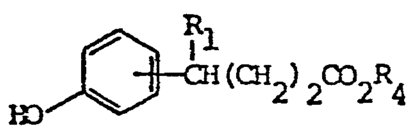

(III)

where $R_4$ is a loweralkyl, to form a compound of the formula

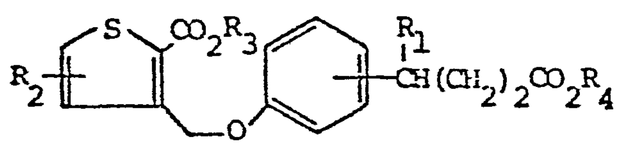

(IV)

Said condensation reaction is conducted, for instance, in the presence of potassium carbonate and potassium iodide in a suitable solvent such as 2-butanone under a reflux condition. Preferred examples of $R_3$ and $R_4$ include methyl and ethyl.

STEP B The diester compound IV is hydrolyzed to afford a novel compound of this invention having the formula

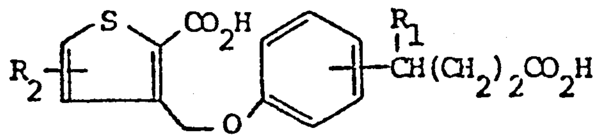

(V)

Said hydrolysis is conducted, for instance, in the presence of water and potassium hydroxide in a suitable solvent such as ethanol under a reflux condition.

STEP C The diacid compound V is cyclized to afford a novel compound of this invention having the formula

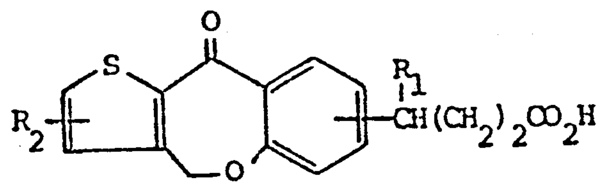

(VI)

Said cyclization is conducted, for instance, in the presence of trifluoroacetic anhydride in a suitable dry solvent such as methylene chloride under a reflux condition.

STEP D-1 The carboxylic acid compound VI is esterified with a compound of the formula ROH where R is loweralkyl or 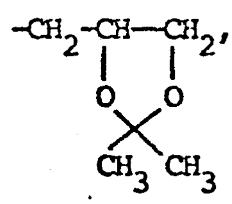, to afford a novel compound of this invention having the formula

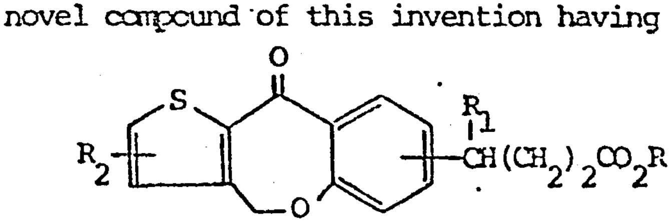

(Ia)

where R is loweralkyl or 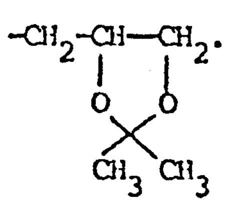

Said esterification is conducted, for instance, in the presence of an acid such as sulfuric acid under a reflux condition. This direct esterification procedure is particularly suitable when ROH is an alcohol of 1-3 carbon atoms, namely, methanol, ethanol or isopropanol.

STEP D-2 As an alternative to Step D-1, the compound VI is first converted to an acid halide and then the latter is reacted with ROH where R is loweralkyl or 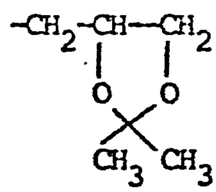 to afford the compound (Ia).

Typically said conversion of the compound VI to the acid halide is conducted by use of an inorganic halide such as, for example, thionyl chloride, phosphorus trihalide or phosphorus pentahalide. The reaction of said acid halide with ROH is conducted, for instance, in a suitable solvent such as diethyl ether or tetrahydrofuran and in the presence of pyridine at ambient temperatures.

STEP E The ketal compound of the formula

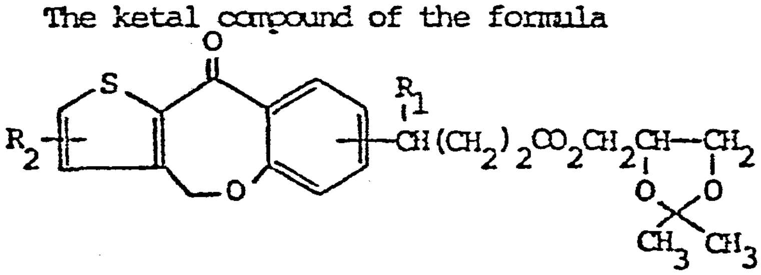

(VII)

is hydrolyzed to afford a compound of the formula

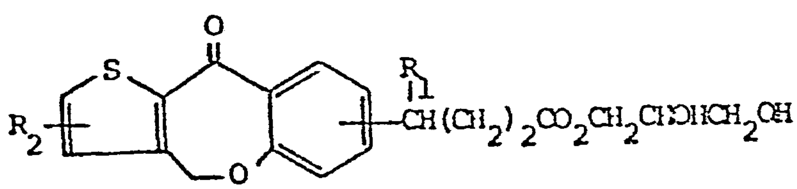

(VIII)

Said hydrolysis is conducted, for instance, in the presence of triethyl borate and dry boric acid ($H_3BO_3$) at an elevated temperature such as 100°C.

The compounds III where $R_1$ is loweralkyl are novel compounds and are prepared in the following manner.

A compound of the formula

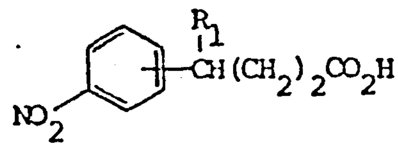

(IX)

is esterified with an alcohol $R_4OH$ where $R_4$ is loweralkyl in a conventional manner to afford a compound of the formula

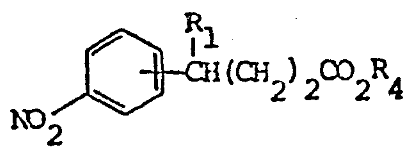

(X)

Compound X is hydrogenated to afford the corresponding amine compound of the formula

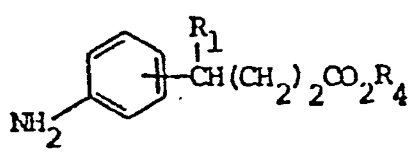

(XI)

Said hydrogenation is conducted in a conventional manner, for instance, by use of a palladium on carbon catalyst in a suitable solvent such as absolute ethanol.

Compound XI is converted to the corresponding diazonium salt in a conventional manner and the latter is hydrolyzed to afford a phenol compound of the formula

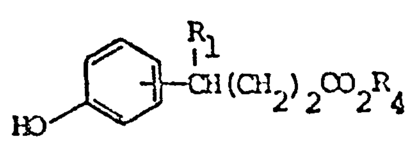

(IIIa)

where $R_1$ is loweralkyl.

Said sequence of diazotization and hydrolysis is conducted, for instance, in the following manner. The amine compound is added dropwise to a cooled mixture (about 1:1) of concentrated sulfuric acid and water while maintaining the cool temperature (i.e. 0-2°C). The mixture is then treated dropwise with an aqueous solution of $NaNO_2$ while maintaining the temperature. The resultant solution is added dropwise to a refluxing aqueous solution of $CuSO_4$ and the reflux is continued for a short period of time, e.g. 10 minutes.

All other starting materials shown above are either known compounds or easily prepared by routine methods known to the art from readily available materials.

The compounds of the present invention are useful as anti-inflammatory agents due to their ability to suppress inflammation in mammals. The activity of the compounds is demonstrated in the carrageenin induced rat paw edema anti-inflammatory assay [Proc. Soc. Exptl. Biol. Med., III 544 (1962), J. Pharmacol. Exp., 141 (1963)]. The results of the anti-inflammatory test of some of the compounds of this invention are given in Table 1.

TABLE 1 - ANTI-INFLAMMATORY DATA

| | $ED_{50}$ (mg/kg;p.o.) | Time Response at 25 mg/kg: % Inhibition | Pretreatment Time |
|---|---|---|---|
| 4-(4,10-Dihydro-10-oxothieno[3,2-c][1]-benzoxepin-8-yl)-butyric acid | 77.9 (60 min) | 49 | 0.5 hr. |
| | | 52 | 1 |
| | | 79 | 2 hrs. |
| | | 94 | 3 |
| | | 95 | 4 |
| | | 67 | 6 |
| | | 47 | 8 |
| | | Time Response at 20 mg/kg: % Inhibition | Pretreatment Time |
| 4-(4,10-Dihydro-10-oxothieno-[3,2-c][1]-benzoxepin-8-yl)-butyric acid methyl ester | 10.3 (6 hrs) | 24 | 2 hrs. |
| | | 54 | 6 |
| | | 62 | 8 |
| | | 16 | 24 |
| | Estimated $ED_{50}$ | Time Response at 20 mg/kg | |
| 4-(4,10-Dihydro-10-oxothieno-[3,2-c]-[1]benzoxepin-8-yl)-butyric acid isopropyl ester | 21.8 (2 hrs) | 31 | 2 hrs. |
| | | 71 | 6 |
| | | 57 | 18 |
| | | 26 | 24 |
| | | Time Response at 20 mg/kg | |
| Ethyl 4-[4,10-dihydro-10-oxothieno-[3,2-c][1]benzoxepin-8-yl]butyrate | --- | 40 | 2 hrs. |
| | | 48 | 6 |
| | | 15 | 18 |
| | | 22 | 24 |

(Table 1 Continued)

| | $ED_{50}$ (mg/kg;p.o.) | Time Response at 20 mg/kg % Inhibition | Pretreating Time |
|---|---|---|---|
| (±)[2,2-Dimethyl-1,3-dioxolan-4-yl]-methyl 4-[4,10-dihydro-10-oxothieno-[3,2-c][1]-benzoxepin-8-yl]butyrate | 14.5 (6 hrs.) | 20 | 2 hrs. |
| | | 61 | 6 |
| | | 32 | 18 |
| | | 41 | 24 |
| | | Time Response at 20 mg/kg | |
| t-Butyl 4-[4,10-dihydro-10-oxothieno-[3,2-c][1]benzoxepin-8-yl]butyrate | --- | 42 | 1 hr. |
| | | 49 | 2 hrs. |
| | | 43 | 4 |
| | | Time Response at 20 mg/kg | |
| (±)-4-(4,10-Dihydro-10-oxothieno[3,2-c]-[1]benzoxepin-8-yl)-pentanoic acid | --- | 25 | 1 hr. |
| | | 23 | 2 hrs. |
| | | 28 | 4 |

The compounds of the invention compare favorably with the well known anti-inflammatory compound sulindac, which, in a similar test, exhibited anti-inflammatory $ED_{50}$ of 10.7 mg/kg p.o.

Compounds of the present invention are also useful as analgesic agents due to their ability to alleviate pain in mammals. The activity of the compounds is demonstrated in the 2-phenyl-1,4-benzoquinone-induced writhing test in mice, a standard assay for analgesia [Proc. Soc. Exptl. Biol. Med., 95, 729 (1957)]. The results of the analgesic activity test of some of the compounds of this invention are given in Table 2.

TABLE 2 - ANALGESIC DATA

| COMPOUND | $ED_{50}$ (p.o.) |
|---|---|
| 4-(4,10-dihydro-10-oxothieno[3,2-c][1]-benzoxepin-8-yl)-butyric acid methyl ester | 10.8 |
| 4-(4,10-Dihydro-10-oxothieno[3,2-c][1]-benzoxepin-8-yl)-butyric acid isopropyl ester. | 8.5 |
| Ethyl 4-[4,10-dihydro-10-oxothieno[3,2-c][1]-benzoxepin-8-yl]butyrate | 8.2 |
| (±)[2,2-Dimethyl-1,3-dioxolan-4-yl]methyl 4-[4,10-dihydro-10-oxothieno[3,2-c][1]benzoxepin-8-yl]butyrate | 11.2 |
| 1-[(4,10-Dihydro-10-oxothieno[3,2-c][1]-benzoxepin-8-yl)-4-butyryl] glyceride | 9.4 |

The compounds of the invention compare favorably with the well known analgesic compound sulindac, which, in a similar test exhibited an analgesic $ED_{50}$ of 6.2 mg/kg p.o.

Effective quantities of the compounds of the invention may be administered to a patient by any of the various methods, for example, orally as in capsules or tablets, parenterally in the form of sterile solutions or suspensions, and in some cases intravenously in the form of sterile solutions. The free acid final products, while effective themselves, may be formulated and administered in the form of their pharmaceutically acceptable addition salts for purposes of stability, convenience of crystallization, increased solubility and the like.

The active compounds of the present invention may be orally administered, for example, with an inert diluent or with an edible carrier, or they may be enclosed in gelatin capsules, or they may be compressed into tablets. For the purpose of oral therapeutic administration, the active compounds of the invention may be incorporated with excipients and used in the form of tablets, troches, capsules, elixirs, suspensions, syrups, wafers, chewing gum and the like. These preparations should contain at least 0.5% of active compound, but may be varied depending upon the particular form and may conveniently be between 4% to about 70% of the weight of the unit. The amount of active compound in such compositions is such that a suitable dosage will be obtained. Preferred compositions and preparations according to the present invention are prepared so that an oral dosage unit form contains between 1.0-300 milligrams of active compound.

The tablets, pills, capsules, troches an the like may also contain the following ingredients: a binder such as micro-crystalline cellulose, gum tragacanth or gelatin; an excipient such as starch or lactose, a disintegrating agent such as alginic acid, Primogel, cornstarch and the like; a lubricant such as magnesium stearate or Sterotex; a glidant such as colloidal silicon dioxide; and a sweetening agent such as sucrose or saccharin may be added or a flavoring agent such as peppermint, methyl salicylate, or orange flavoring. When the dosage unit form is a capsule, it may contain, in addition to materials of the above type, a liquid carrier such as a fatty oil. Other dosage unit forms may contain other various materials which modify the physical form of the dosage unit, for example, as coatings. Thus tablets or pills may be coated with sugar, shellac, or other enteric coating agents. A syrup may contain, in addition to the active compounds, sucrose as a sweetening agent and certain preservatives, dyes, colorings and flavors. Materials used in preparing these various compositions should be pharmaceutically pure and non-toxic in the amounts used.

For the purposes of parenteral therapeutic administration, the active compounds of the invention may be incorporated into a solution or suspension. These preparations should contain at least 0.1% of active compound, but may be varied to be between 0.5 and about 30% of the weight thereof. The amount of active compound in such compositions is such that a suitable dosage will be obtained. Preferred compositions and preparations according to the present invention are prepared so that a parenteral dosage unit contains between 0.5 to 100 milligrams of active compound.

The solutions or suspension may also include the following components: a sterile diluent such as water for injection, saline solution, fixed oils, polyethylene glycols, glycerine, propylene glycol or other synthetic solvents; antibacterial agents such as benzyl alcohol or methyl parabens; antioxidants such as ascorbic acid or sodium bisulfite; chelating agents such as ethylenediaminetetraacetic acid; buffers such as acetates, citrates or phosphates and agents for the adjustment of tonicity such as sodium chloride or dextrose. The parenteral preparation can be enclosed in disposable syringes or multiple doze vials made of glass or plastic.

Examples of the compounds of this invention include:

4-(4,10-dihydro-10-oxothieno[3,2-c][1]benzoxepin-8-yl)-butyric acid;

4-(4,10-dihydro-10-oxothieno[3,2-c][1]benzoxepin-8-yl)-butyric acid methyl ester;

ethyl 4-[4,10-dihydro-10-oxothieno[3,2-c][1]benzoxepin-8-yl]butyrate;

4-(4,10-dihydro-10-oxothieno[3,2-c][1]benzoxepin-8-yl)-butyric acid isopropyl ester;

t-butyl 4-[4,10-dihydro-10-oxothieno[3,2-c][1]benzoxepin-8-yl]butyrate;

(±)[2,2-dimethyl-1,3-dioxolan-4-yl]methyl 4-[4,10-dihydro-10-oxothieno-[3,2-c][1]benzoxepin-8-yl]butyrate;

1-[(4,10-dihydro-10-oxothieno[3,2-c][1]benzoxepin-8-yl)-4-butyryl] glyceride; and

4-(4,10-dihydro-10-oxothieno[3,2-c][1]benzoxepin-8-yl)pentanoic acid;

which belong to compounds of formula I; and intermediate compounds

4-(2-carboxy-3-thienyl)methoxyphenylbutyric acid;

ethyl 4-(4-nitrophenyl)pentanoate;

ethyl 4-(4-aminophenyl)pentanoate;

ethyl 4-(4-hydroxyphenyl)pentanoate;

ethyl 4-[4-(2-carbomethoxy-3-thienyl)methoxyphenyl]pentanoate; and

4-[4-(2-carboxy-3-thienyl)methoxyphenyl]pentanoic acid.

The following examples are for illustrative purposes and are not to be considered as limiting the invention disclosed herein. All temperatures are given in degrees Celcius.

## EXAMPLE 1

4-(2-Carboxy-3-thienylmethoxyphenyl)butyric acid

A mixture of 12.42g (0.053 m) of 3-bromomethyl-2-carbomethoxy thiophene, 11g (0.053 m) of ethyl p-hydroxyphenylbutyrate, 29g (0.212 m) of $K_2CO_3$ and 0.53g of potassium iodide in 250 ml of 2-butanone was refluxed 5 hours. The salts were filtered off and rinsed with ether. The filtrate was concentrated in vacuo to an oil. The oil was dissolved in ether, washed with 5% NaOH and water, dried over $Na_2SO_4$, filtered and concentrated in vacuo to give 18.9g (98%) of the diester.

A mixture of 18g (0.05 m) of the diester, 41g (0.74 m) of potassium hydroxide, 33 ml of $H_2O$ and 264 ml of ethanol was refluxed overnight. The reaction mixture was concentrated in vacuo to give a salt which dissolved in water. The aqueous layer was extracted with ether, cooled and acidified with concentrated HCl. The precipitate was collected and dried giving 14g of crude material. Recrystallization from isopropanol afforded 9g (56%) of product, m.p. 147-151°C.

ANALYSIS:

| | | |
|---|---|---|
| Calculated for $C_{16}H_{16}O_5S$: | 59.99%C | 5.03%H |
| Found: | 59.60%C | 5.18%H |

EXAMPLE 2

4-(4,10-Dihydro-10-oxothieno[3,2-c][1]benzoxepin-8-yl)-butyric acid

A stirred suspension of 10.2g (0.0318 mol) of 4-(2-carboxy-3-thienyl)methoxyphenylbutyric acid and 100 ml of dry $CH_2Cl_2$ was treated dropwise over 30 seconds with 14.91g (0.071 mol) of trifluoroacetic anhydride. The resultant solution was heated under reflux with stirring for approximately 15 hours. After cooling to ambient temperature, the solution was treated with 70 ml of water and 50 ml of 5% hydrochloric acid solution. The organic phase was washed once with water, dried over $Na_2SO_4$, and concentrated to an oil which was further dried by azeotropic distillation of toluene. The crude crystalline product was recrystallized from 30 ml of toluene to afford 7.02g (73.0%) of crystals, m.p. 107-109.5°C.

ANALYSIS:

| | | |
|---|---|---|
| Calculated for $C_{16}H_{14}O_4S$: | 63.56%C | 4.67%H |
| Found: | 63.24%C | 4.62%H |

0113867

EXAMPLE 3

4-(4,10-Dihydro-10-oxothieno[3,2-c][1]benzoxepin-8-yl)-butyric acid methyl ester

A mixture of 7g (0.023 m) of 4-(4,10-dihydro-10-oxothieno[3,2-c][1]benzoxepin-8-yl)-butyric acid, 5 ml of methanol and 4 drops of concentrated $H_2SO_4$ was refluxed 3 hours. The mixture was diluted with water and extracted with ether. The ether extract was washed with 5% $Na_2CO_3$, water, dried over $Na_2SO_4$, filtered and evaporated to give 7.00g (90%) of an oil.

ANALYSIS:

| | | |
|---|---|---|
| Calculated for $C_{17}H_{16}O_4S$: | 64.54%C | 5.10%H |
| Found: | 64.46%C | 5.02%H |

EXAMPLE 4

4-(4,10-Dihydro-10-oxothieno[3,2-c][1]benzoxepin-8-yl)-butyric acid isopropyl ester

A mixture of 7g (0.023 m) of 4-(4,10-dihydro-10-oxothieno-[3,2-c][1]benzoxepin-8-yl)-butyric acid, 5 ml of isopropyl alcohol and four drops of concentrated $H_2SO_4$ was refluxed three hours. After standing overnight at ambient temperature the mixture was diluted with water and extracted with ether. The ether extract was washed with 5% $Na_2CO_3$, water, dired over $Na_2SO_4$, filtered and evaporated to give 5.69g (71%) of an oil.

ANALYSIS:

| | | |
|---|---|---|
| Calculated for $C_{19}H_{20}O_4S$: | 66.26%C | 5.85%H |
| Found: | 66.37%C | 5.84%H |

## EXAMPLE 5

Ethyl 4-[4,10-dihydro-10-oxothieno[3,2-c][1]benzoxepin-8-yl]butyrate

To a solution of 7g (0.023 m) of 4-(4,10-dihydro-10-oxothieno-[3,2-c][1]benzoxepin-8-yl)-butyric acid in 5 ml of absolute EtOH, four drops of concentrated sulfuric acid was added. The mixture was refluxed for 3 hours and allowed to stand overnight at ambient temperature. The reaction mixture was diluted with water and extracted with ether. The ether extract was washed with 5% $Na_2CO_3$, dried over $Na_2SO_4$, filtered and evaporated to give 6.13g (81%) of an oil.

ANALYSIS:

| | | |
|---|---|---|
| Calculated for $C_{18}H_{18}O_4S$: | 65.44%C | 5.49%H |
| Found: | 65.59%C | 5.45%H |

## EXAMPLE 6

t-Butyl 4-[4,10-dihydro-10-oxothieno[3,2-c][1]benzoxepin-8-yl]butyrate

To a solution of 3.0g (0.01 m) of 4-(4,10-dihydro-10-oxothieno[3,2-c][1]benzoxepin-8-yl)-butyric acid in 50 ml of $CH_2Cl_2$, 1.31g (0.011 m) of $SOCl_2$ was added dropwise and the mixture was refluxed two hours. The solvent was evaporated and the acid chloride structure was confirmed by IR.

Under an atmosphere of nitrogen, 37 ml of t-butanol was kept at room temperature while 5.7 ml of n-butyl lithium in hexane (2.2 m) was added dropwise. After stirring for 15 minutes, a solution of the acid chloride in anhydrous ether was added dropwise. The resulting slurry was stirred overnight. The suspension was partitioned between water and a mixture of ether/ethyl acetate. The organic layer was washed with saturated NaCl solution, dried over $Na_2SO_4$, filtered and evaporated to give 1.2g (34%) of an oil.

ANALYSIS:

| | | |
|---|---|---|
| Calculated for $C_{20}H_{22}O_4S$: | 67.01%C | 6.19%H |
| Found: | 66.79%C | 6.15%H |

EXAMPLE 7

(±)[2,2-dimethyl-1,3-dioxolan-4-yl]methyl 4-[4,10-dihydro-10-oxothieno-[3,2-c][1]benzoxepin-8-yl]butyrate.

To a solution of 8.77g (0.029 m) of 4-(4,10-dihydro-10-oxothieno[3,2-c][1]benzoxepin-8-yl)-butyric acid in 50 ml of $CH_2Cl_2$, 3.8g (0.03 m) of $SOCl_2$ was added dropwise. The mixture was stirred 0.5 hour at ambient temperature, then just brought to reflux. The reaction mixture was evaporated giving the acid chloride as an oil.

A solution of the acid chlorie in 100 ml of THF was added dropwise to a stirred solution of (±)-2,2-dimethyl-1,3-dioxolane-4-methanol (Solketal) in 100 ml of THF. The mixture was stirred overnight. To the mixture 2.37g (0.03 m) of pyridine was addded. The salt which formed was filtered off. The filtrate was evaporated and the residue dissolved in ether and washed with 5% $Na_2CO_3$. The ether extract was dried over $Na_2SO_4$, filtered and evaporated to afford 6g (60%) of an oil.

ANALYSIS:

| | | |
|---|---|---|
| Calculated for $C_{22}H_{24}O_6S$: | 63.45%C | 5.81%H |
| Found: | 63.22%C | 5.76%H |

EXAMPLE 8

1-[(4,10-Dihydro-10-oxothieno[3,2-c][1]benzoxepin-8-yl)-4-butyryl] glyceride

To a mixture of 3.25g (0.0078 m) of (±)[2,2-dimethyl-1,3-dioxolan-4-yl]methyl 4-[4,10-dihydro-10-oxothieno[3,2-c][1]benzoxepin-8-yl]butyrate and 50 ml of triethyl borate, 1.2g of dry $H_3BO_3$ was added. The reaction mixture was heated to 100°C for 10 minutes. The solvent was removed under vacuum at 100° over 30 minutes. The residue was partitioned between ether and water. The aqueous layer was extracted several times with ether. The combined ether extracts were washed with water, dried over $Na_2SO_4$, filtered and evaporated to give an oil. The oil was titurated with pentane. The pentane was decanted. The oil was dried in vacuo to afford 2.6g (90%) of product.

ANALYSIS:

| | | |
|---|---|---|
| Calculated for $C_{19}H_{20}O_6S$: | 64.54%C | 5.10%H |
| Found: | 64.46%C | 5.02%H |

## EXAMPLE 9

Ethyl (±)-4-(4-nitrophenyl)pentanoate

A mixture of 110 g (0.49 m) of (±)-4-(p-nitrophenyl)valeric acid[1], 660 ml of absolute ethanol and 10 ml of concentrated sulfuric acid was refluxed overnight. The ethanol was evaporated in vacuo and the residue was partitioned between ether and water. The ether extract was washed with 5% $NaHCO_3$, and then washed with water, dried over $Na_2SO_4$, filtered and evaporated to give 119 g (97%) of pure product. The product could be distilled at 127-128°C (1.5 mm).

ANALYSIS:

| | | | |
|---|---|---|---|
| Calculated for $C_{13}H_{17}NO_4$: | 62.14%C | 6.82%H | 5.58%N |
| Found: | 62.28%C | 6.92%H | 5.45%N |

[1] J. Colonge and E. Fichet, Bull. Soc. Chim. Fr., 412 (1955).

## EXAMPLE 10

Ethyl (±)-4-(4-aminophenyl)pentanoate.

A mixture of 114 g (0.45 m) of ethyl (±)-4-(4-nitrophenyl) pentanoate, 170 ml of absolute ethanol and 2 g of 10% Pd/C was hydrogenated (Paar shaker) until consumption of hydrogen ceased. The catalyst was filtered off and the ethanol evaporated. The residue was partitioned between water and ether. The ether extract was washed with 5% $NaHCO_3$ and water. The ether extract was dried over $Na_2SO_4$, filtered and evaporated to give the product as an oil. The oil was distilled at 118-122°C (0.55-0.60 mm) giving 85 g (86%) of pure product.

ANALYSIS:

| | | | |
|---|---|---|---|
| Calculated for $C_{13}H_{19}NO_2$: | 70.56%C | 8.65%H | 6.33%N |
| Found: | 70.69%C | 8.64%H | 6.18%N |

EXAMPLE 11

Ethyl (±)-4-(4-hydroxyphenyl)pentanoate

To a cooled (0°C) solution of 350g concentrated sulfuric acid and 350 ml of water, 40g (0.18 m) of ethyl (±)-4-(4-aminophenyl) pentanoate was added dropwise maintaining the temperature at 0-2°C. The mixture was then treated dropwise with 13.66g (0.198 m) of $NaNO_2$ in 25 ml of water at such a rate that the temperature did not exceed 2°C. The resultant solution was added dropwise to a refluxing solution of 387g (2.43 m) of $CuSO_4$ in 600 ml of water over a period of approximately 30 minutes. The mixture was refluxed an additional 10 minutes. The mixture was cooled to room temperature and 800 ml of water was added. The mixture was extracted thrice with $CH_2Cl_2$ (additional water was needed to dissolve the salts.) The organic phase was washed with water, dried over $Na_2SO_4$, filtered and evaporated to give 29.6g of an oil. The oil was distilled at 129°C (0.6 mm) and the distillate solidified on cooling to give 21.05g (53%) of pure product, m.p. 32-34°C.

ANALYSIS:

| | | |
|---|---|---|
| Calculated for $C_{13}H_{18}O_3$: | 70.25%C | 8.16%H |
| Found: | 69.98%C | 8.19%H |

EXAMPLE 12

Ethyl (±)-4-[4-(2-carbomethoxy-3-thienyl)methoxyphenyl]pentanoate

A stirred mixture of ethyl (±)-4-(4-hydroxyphenyl)pentanoate (5.56g, 0.025 mol), 3-bromomethyl-2-carbomethoxythiophene (5.88g, 0.025 mol), anhydrous potassium carbonate (10.37g, 0.075 mol), potassium iodide (0.15g) and 2-butanone (85 ml) was heated overnight under reflux with exclusion of moisture. The mixture was vacuum filtered hot and the filter cake was washed with 2-butanone. The combined filtrate was concentrated to an oil which was dissolved in dichloromethane (300 ml). The solution was washed with 10% sodium hydroxide, water and saturated sodium chloride solution. The dried ($Na_2SO_4$) organic phase was concentrated to an oil (9.0g) which was subjected to HPLC purification ( silica gel column, 10% ethyl acetate in hexane, UV detector) to afford after concentration of the product-containing fractions 7.37g of an oil. The oil was subjected to further concentration in vacuo at 60°C to give 7.13g (75.8%) of an oil.

ANALYSIS:

| | | |
|---|---|---|
| Calculated for $C_{20}H_{24}O_5S$: | 63.81%C | 6.43%H |
| Found: | 63.69%C | 6.50%H |

0118867

EXAMPLE 13

(±)-4-[4-(2-Carboxy-3-thienyl)methoxyphenyl]pentanoic acid

A stirred mixture of ethyl (±)-4-[4-(2-carbomethoxy-3-thienyl)methoxyphenyl]pentanoate (18.79g, 0.05 mol), potassium hydroxide (28.06g, 0.5 mol), water (30 ml) and 95% ethanol (180 ml) was refluxed for four hours and then allowed to stand overnight at ambient temperature. The solution was diluted with water and concentrated on a rotary evaporator to remove most of the alcohol. Chipped ice was added and the mixture was acidified with concentrated hydrochloric acid to afford a suspension. The aqueous phase was decanted and the residue was washed with water to afford a semisolid. A solution of the semisolid and ether (300 ml) was dried over $Na_2SO_4$, filtered and concentrated to afford an oil which crystallized on standing at 5°C. The solid was triturated with hexane and isolated by vacuum filtration to give a crude product (15.36g). Recrystallization from acetonitrile (40 ml) afforded 13.23g (79.1%) of crystals, m.p. 137.5-139.5°C

ANALYSIS:

| | | |
|---|---|---|
| Calculated for $C_{17}H_{18}O_5S$: | 61.06%C | 5.42%H |
| Found: | 61.12%C | 5.48%H |

## EXAMPLE 14

(±)-4-(4,10-Dihydro-10-oxothieno[3,2-c][1]benzoxepin-8-yl)pentanoic acid

A stirred suspension of (±)-4-[4-(2-carboxy-3-thienyl)methoxyphenyl]pentanoic acid (10.84g, 0.0324 mol) and dichloromethane (100 ml) was treated in one portion with trifluoroacetic anhydride (15.88g, 0.0756 mol). On continued stirring over a few minutes the solid dissolved to afford a solution which was heated under reflux for 4 hours. The cooled solution was treated with water and stirred for 20 minutes at ambient temperature. The phases were separated and the organic phase was washed with water, dried over $Na_2SO_4$, filtered and concentrated to an oil. A sample of the oil was triturated with toluene to afford a solution which was diluted with hexane whereupon the product separated as an oil which slowly crystallized. Thin layer analysis (silica gel, ethyl acetate) of the oil indicated two impurities with higher $R_f$ values than the product. A solution of the oil and dichloromethane (100 ml) was extracted with 1.3% sodium hydroxide solution (100 ml) and the aqueous phase was extracted with a little dichloromethane. The aqueous phase was acidified with concentrated hydrochloric acid and extracted with dichloromethane (2X 100 ml). The combined, dried ($Na_2SO_4$) organic phase was concentrated to an oil which was cooled, seeded with the crystals obtained above and triturated with hexane to give 7.26g (70.8%) of the product as a solid, m.p. 83-86°C.

ANALYSIS:

| | | |
|---|---|---|
| Calculated for $C_{17}H_{16}O_4S$: | 64.54%C | 5.10%H |
| Found: | 64.59%C | 5.16%H |

CLAIMS:

1. A 4,10-dihydro-10-oxothieno benzoxepin derivative having the formula

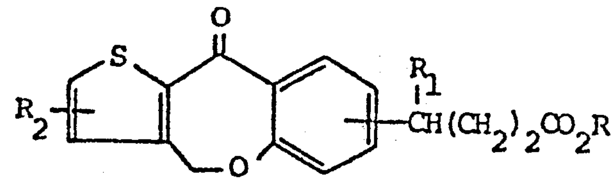

where R is hydrogen, loweralkyl, $-CH_2CH(OH)CH_2OH$, or 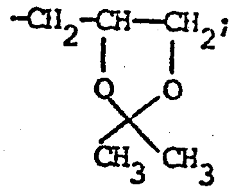 ; $R_1$ is hydrogen or loweralkyl; and $R_2$ is hydrogen or loweralkyl, or where appropriate the optical isomers thereof or the racemic mixture thereof.

2. The compound as defined in Claim 1 wherein $R_2$ is hydrogen.

3. The compound as defined in Claim 1 wherein $R_1$ is hydrogen.

4. The compound as defined in Claim 1 wherein $R_1$ is methyl.

5. The compound as defined in Claim 1, which is 4-(4,10-dihydro-10-oxothieno[3,2-c][1]benzoxepin-8-yl)pentanoic acid, the optical isomers thereof or the racemic mixture thereof.

6. The compound as defined in Claim 1, which is 4-(4,10-dihydro-10-oxothieno [3,2-c] [1]benzoxepin-8-yl)-butyric acid methyl ester.

7. The compound as defined in Claim 1, which is 4-(4,10-dihydro-10-oxothieno [3,2-c] [1] benzoxepin-8-yl)-butyric acid isopropyl ester.

8. The compound as defined in Claim 1 wherein R is 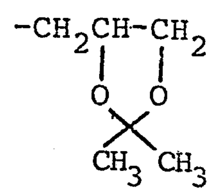

9. The compound as defined in Claim 8, which is (2,2-dimethyl-1,3-dioxolan-4-yl)methyl 4-(4,10-dihydro-10-oxothieno-[3,2-c]-[1]benzoxepin-8-yl)butyrate, the optical isomers thereof or the racemic mixture thereof.

10. A compound having the formula

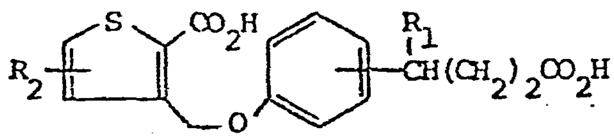

where $R_1$ and $R_2$ are each independently hydrogen or loweralkyl, or where appropriate, the optical isomers thereof or the racemic mixture thereof.

11. A pharmaceutical composition comprising as active ingredient a compound of the formula I as defined in claim 1 or one or both the optical isomers thereof or the racemic mixture thereof in association with a pharmaceutically acceptable carrier and/or stabilizer.

12. A compound of the formula I as claimed in claim 1 for use as a medicament.

13. A method of preparing a compound of the formula I

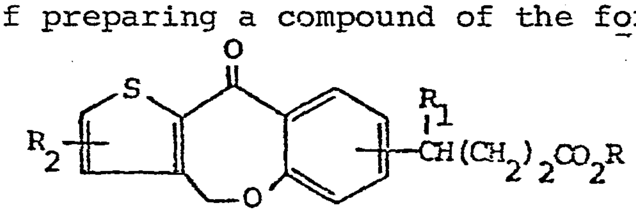

where $R_1$ and $R_2$ are each independently hydrogen or lower alkyl * which comprises cyclizing a compound of the formula V

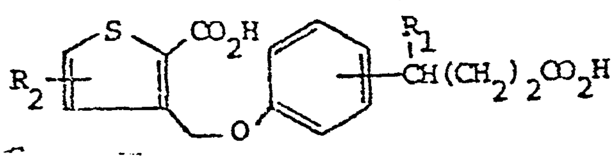

where $R_1$ and $R_2$ are each independently hydrogen or lower alkyl, to afford a compound of the formula I

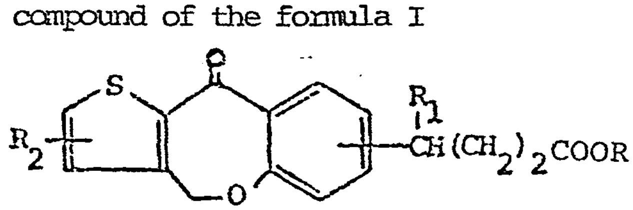

* and R is as defined in claim 1

where R is hydrogen,

optionally esterifying the compound obtained with a compound of the formula ROH where R is loweralkyl or

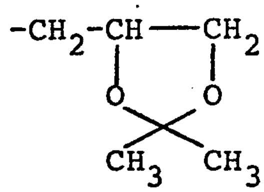

to afford a compound of the formula I where R is as defined, or optionally first converting a compound of the formula I where R is hydrogen to an acid halide and then reacting the latter with ROH where R is loweralkyl or

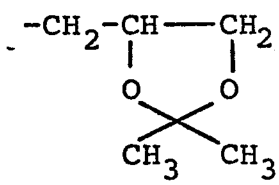

to afford a compound of the formula I where R is as defined, and

optionally hydrolyzing a compound of the formula I where R is

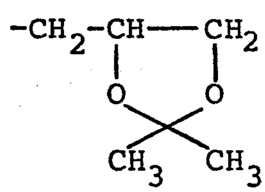

to afford a compound of the formula I where R is $-CH_2CH(OH)CH_2OH$.

14. The method as claimed in claim 13, wherein the cyclization is conducted in the presence of a dehydrating agent.

15. The method as claimed in claim 14 wherein the dehydrating agent is trifluoroacetic anhydride.

16. The method as claimed in claims 13-15 wherein $R_2$ is hydrogen.

17. The method as claimed in claims 13-15 wherein $R_1$ is hydrogen.

18. The method as claimed in claims 13-15 wherein $R_1$ is methyl.

0118867
HOE 83/S 007

Claims for Austria

1. A method of preparing a compound of the formula

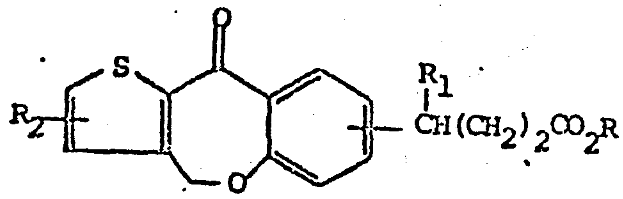

(I)

where R is hydrogen, loweralkyl, $-CH_2CH(OH)CH_2OH$ or

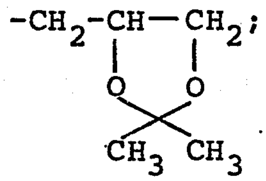

; $R_1$ is hydrogen or loweralkyl, and $R_2$ is hydrogen or loweralkyl, or where appropriate the optical isomers thereof or the racemic mixture thereof, which comprises cyclizing a compound of the formula V

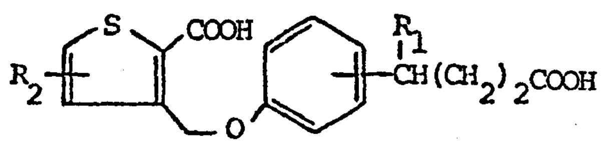

(V)

where $R_1$ and $R_2$ are as defined above to afford a compound of the formula I

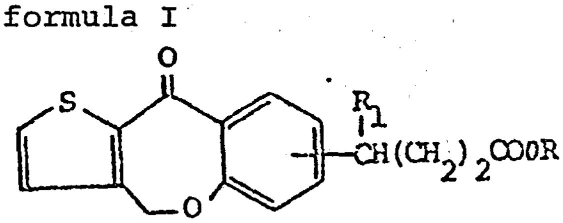

where R is hydrogen,

optionally esterifying the compound obtained with a compound of the formula ROH where R is loweralkyl or

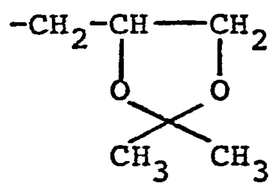

to afford a compound of the formula I where R is as defined, or optionally first converting a compound of the formula I where R is hydrogen to an acid halide and then reacting the latter with ROH where R is loweralkyl or

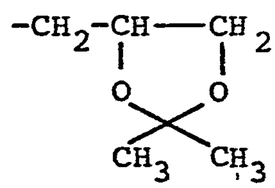

to afford a compound of the formula I where R is as defined, and

optionally hydrolyzing a compound of the formula I where R is

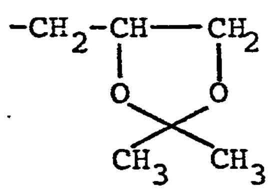

to afford a compound of the formula I where R is $-CH_2CH(OH)CH_2OH$.

2. The method as claimed in claim 1, wherein the cyclization is conducted in the presence of a dehydrating agent.

3. The method as claimed in claim 2, wherein the dehydrating agent is trifluoroacetic anhydride.

4. The method as claimed in claims 1-3, wherein $R_2$ is hydrogen.

5. The method as claimed in claims 1-3, wherein $R_1$ is hydrogen.
6. The method as claimed in claims 1-3, wherein $R_1$ is methyl.